# EUROPÄISCHE PATENTANMELDUNG

(11) **EP 2 255 760 A2**
(43) Veröffentlichungstag der Anmeldung: **01.12.2010**
(21) Anmeldenummer: 10152328.0
(22) Anmeldetag: 11.08.2000
(51) Int. Cl.: A61F 9/01

(54) **Verfahren und Vorrichtung zur vollständigen Korrektur von Sehfehlern des menschlichen Auges**

(30) Priorität: 11.08.1999 DE 19938203
(62) Teilanmeldung aus: 00951495.1
(71) Anmelder: Carl Zeiss Meditec AG, 07745 Jena (DE)
(72) Erfinder: Dick, Manfred, 07926, Gefell (DE); Schröder, Eckhard, 90542, Eckental (DE); Fiedler, Joachim, 74564, Crailsheim (DE)
(74) Vertreter: Breit, Ulrich

(57) **Zusammenfassung**

Die Erfindung betrifft ein Verfahren und eine Vorrichtung zur vollständigen Korrektur von Sehfehlern des menschlichen Auges. Es wurden Kombinationen von Meß- und Bearbeitungsverfahren angegeben, welche in ihrer erfindungsgemäßen Anwendung die vollständige Korrektur des menschlichen Auges ermöglichen. Dabei werden Meßverfahren eingesetzt, welche die Oberfläche der Cornea präzise erfassen können und auch die im weiteren Strahlengang bis zur Netzhaut entstehenden Abbildungsfehler registrieren. Die rechnergestützte Auswertung dieser Meßergebnisse ergibt in Verbindung mit der Berechnung ideal korrigierter Augenlinsen (beispielsweise nach Katarakt-Operationen) oder ideal korrigierender Corneaoberflächen die Möglichkeit, bevorzugt mit einem Spot-Scanning-Excimerlasersystem topographiegestützt eine patientenspezifische Linse herzustellen und/oder die Hornhaut ideal korrigierend zu formen.

## Beschreibung

Die Erfindung betrifft ein Verfahren und eine Vorrichtung zur Korrektur von Sehfehlern des menschlichen Auges.

In der Ophthalmologie ist es bekannt, die Hornhaut bei Sehschwäche durch Ablation von Gewebe zu formen. Die Daten über die Aberration im Strahlengang des Auges werden dabei über eine Befragung des Patienten aufgrund von Korrekturen über standardisierte Korrekturlinsen vor dem Auge des Patienten nach seinem subjektiven Eindruck des Sehvermögens gewonnen. Daneben existieren Verfahren zur Vermessung der äußeren Kontur des Auges mittels Streifen- oder Ringprojektionssystemen, wie sie beispielsweise von den Firmen Orbtek, Tomey oder Technomed hergestellt werden.

In der DE 197 05 119 A1 wird ein Verfahren zur Verbesserung eines Shack-Hartmann-Sensors beschrieben, mit dem Wellenfronten im Bereich der Astronomie zur Vermessung von Sternen gemessen werden können.

In der DE 197 27 573 C1 wird in einem wertvollen Beitrag zum Stand der Technik eine Vorrichtung und ein Verfahren zur Formgebung von Oberflächen, insbesondere von Linsen, vermittels einer Laserabtragung der Oberflächen angegeben.

Nachteilig am Stand der Technik wird die Tatsache empfunden, daß die Korrektur der Linsen nur aufgrund suboptimaler Daten über die Ursachen der Sehfehler wie Irregularitäten der Hornhautoberfläche oder Aberration im Strahlengang stattfindet. Es werden folglich nur Korrekturen entsprechend den Standardlinsenformeln der geometrischen Optik ausgeführt.

Aufgabe der vorliegenden Erfindung war es daher, ein Verfahren und eine Vorrichtung bereitzustellen, die eine vollständige Korrektur aller refraktiven Sehfehler einschließlich der Aberrationen des Strahlenganges im fehlsichtigen Auge erlauben.

Diese Aufgabe wird durch die Vorrichtung und das Verfahren nach den unabhängigen Ansprüchen gelöst. Vorteilhafte Ausgestaltungen der Erfindung sind in den Unteransprüchen angegeben.

Insbesondere wird die Aufgabe durch eine Vorrichtung zur Korrektur von Sehfehlern eines Auges gelöst, umfassend eine kohärente Lichtquelle, eine Strahlmodifikationseinrichtung zur Formung und Ablenkung eines Strahles der kohärenten Lichtquelle, wobei eine Wellenfrontanalyseeinrichtung zur Analyse einer Wellenfront des Strahlenganges im Auge vorgesehen ist. Durch diese Vorrichtung ist es möglich, die aus der Analyse der intraokularen Aberration gewonnenen Daten in die Korrektur eines bestehenden optischen Systems eines zu korrigierenden Auges einfließen zu lassen. Damit wird die Korrektur des optischen Systems des Auges nochmals präziser möglich.

Als Auge kommt insbesondere ein menschliches Auge in Betracht, denkbar ist aber auch die Korrektur von Augen anderer Lebewesen. Sehfehler sind insbesondere refraktive Sehfehler wie die Kurz- oder Weitsichtigkeit, Irregularitäten der Hornhautoberfläche oder Aberrationen im Strahlengang.

Als kohärente Lichtquelle ist bevorzugt ein Laser, besonders bevorzugt ein refraktiver Laser, insbesondere bevorzugt ein Spot-Scanning-Excimerlasersystem, vorgesehen. Daneben kann auch an einen Spot-Scanner mit Laserlicht in anderen Bereichen des Spektrums gedacht werden, wie einen frequenzverfünffachten YAG-Laser, einen IR-Laser bei 3 µm, wie bspw. einen Erbium:YAG Laser, der bei 2,94 µm emittiert, oder einen femto Sekundenlaser (FS-Laser).

Die Strahlenmodifikationseinrichtung besteht bevorzugt aus einer Einrichtung zur Formung eines Strahles und einer Einrichtung zur Ablenkung und Ausrichtung des Strahles. Als Einrichtung zur Formung des Strahles werden bevorzugt Linsensysteme, diffraktive Strukturen und refraktive Elemente eingesetzt. Als Einrichtung zur Ablenkung und Ausrichtung des Strahles werden bevorzugt Scanneranordnungen, Prismen und Spiegel verwendet.

Als Wellenfrontanalyseeinrichtung kann bevorzugt ein Shack-Hartmann-Sensor verwendet werden. Hierbei handelt es sich um einen Sensor, der auf einem Verfahren beruht, um Wellenfronten zu analysieren. Er wird insbesondere in der Astronomie eingesetzt (s. o.). Durch diese Wellenfrontanalyseeinrichtung kann die gesamte aus dem Auge austretende Wellenfront gemessen werden, und so können Informationen über die Sehfehler einschließlich der intraokularen Aberration des Strahlenganges auch im Auge gewonnen werden.

Bei einem weiteren Ausführungsbeispiel der vorliegenden Erfindung ist eine Vorrichtung vorgesehen, bei der zusätzlich eine Topographieanalyseeinheit zur Analyse der Oberfläche des Auges vorgesehen ist. Diese Analyse liefert die Information, welche Krümmung und Kontur die Augenoberfläche - also insbesondere die Cornea - aufweist. Dadurch stehen dem System die vollständigen Daten über die refraktiven Sehfehler des Auges zur Verfügung. Sowohl die gegebenenfalls nicht ideale Oberflächenkontur des Auges - bzw. der Cornea - als auch die intraokulare Aberration können nun analysiert werden und stehen dem System bei der Korrektur des optischen Systems des Auges zur Verfügung. Dadurch ist es möglich, die Sehfehler des Auges vollständig zu korrigieren und sogar ein Sehvermögen zu erreichen, das über dem des normalen menschlichen Auges liegt.

Bei einem weiteren Ausführungsbeispiel der Erfindung ist eine Vorrichtung vorgesehen, bei der weiter eine Steuereinheit zur Verarbeitung von Signalen der Wellenfrontanalyseneinheit und/oder zur Verarbeitung von Signalen der Topographieanalyseeinheit, und/oder zur Steuerung der kohärenten Lichtquelle und/oder zur Steuerung der Strahlmodifikationseinrichtung vorgesehen ist. Durch diese Steuereinheit können die durch die Analyseeinheiten ermittelten Daten ausgewertet werden. Es ist möglich, die Signale der Wellenfrontanalyseneinheit und der Signale der Topographieanalyseeinheit in der Steuereinheit getrennt zu verarbeiten und auszuwerten oder beide Datenmengen in einem Schritt zu verarbeiten. Die Steuereinheit besteht bevorzugt aus mehreren einzelnen Steuereinheiten.

Diese Daten dienen bevorzugt der Bereitstellung eines idealen optischen Systems. Aus diesen Daten werden die für die Strahlmodifikation erforderlichen Parameter bestimmt. Diese Parameter können bevorzugt in einem weiteren Schritt zur Steuerung der kohärenten Lichtquelle benutzt werden, um beispielsweise Amplitude, Pulsdauer und Energie des Strahles vorzubestimmen. Weiter bevorzugt werden diese Parameter auch zur Steuerung der Strahlmodifikationseinrichtung eingesetzt, um hier über die Ablenkung des Strahles den Zielort und die Geometrie des Strahles im Ziel festzulegen.

Dadurch können bei einem bevorzugten Ausführungsbeispiel insbesondere die Schußpositionen für die Herstellung der einzelnen Elemente berechnet werden.

Bei einem weiteren bevorzugten Ausführungsbeispiel der vorliegenden Erfindung ist eine Vorrichtung vorgesehen, bei der die Strahlmodifikationseinrichtung so ausgebildet ist, daß mit dem Strahl eine Intraokularlinse und/oder eine Augenlinse und/oder die Cornea des Auges und/oder eine Kontaktlinse und/oder eine implantable Contact lens (ICL) und/oder ein Brillenglas bearbeitbar ist. Durch den bevorzugt von der Steuereinheit gesteuerten Strahl kann nun ein Element bzw. Werkstück des Linsensystems derart bearbeitet werden, daß die Sehfehler bzw. Aberration vollständig korrigiert wird. Ein solches Element ist bevorzugt eine Intraokularlinse (IOL), die vor einer entsprechenden Operation vorgefertigt wird. Besonders bevorzugt handelt es sich um eine ICL (implantable contact lens), die auf die Linse aufgesetzt wird. Diese IOL bzw. ICL kann dann aufgrund der gesamten vorliegenden Information aber die Sehfehler einschl. der Aberration des Auges so geformt werden, daß sie alle vorhandenen Sehfehler korrigiert. Denkbar ist auch, die Korrektur mittels des bevorzugt durch die Steuereinrichtung gesteuerten Strahles an der Augenlinse selbst vorzunehmen.

Weiter ist es denkbar, eine Korrektur durch die Bearbeitung der Cornea vorzunehmen. Bevorzugt werden auch Kontaktlinsen gefertigt, die patientenspezifisch sämtliche individuellen, über dem refraktiven Augenfehler hinausgehende Fehler wie Aberrationen, unsymmetrische Zylinder- und Hornhaut-Irregularitäten korrigieren. Daneben ist es möglich, individuelle Brillengläser herzustellen. Hierfür können neben der Excimer-Spot-Bearbeitung auch Methoden der Optikindustrie wie beispielsweise das single point diamond turning Verfahren eingesetzt werden. Hierdurch können sämtliche Elemente des betroffenen optischen Systems zur Korrektur der Augenfehler verwendet werden.

Es ist ebenfalls möglich, eine Kombination der einzelnen (teil-)korrigierten Elemente einzusetzen. Dies ist insbesondere dann vorteilhaft, wenn die theoretisch mögliche Korrektur über ein Element zu einer hohen Beanspruchung dieses Elements führen würde und eine solche Beanspruchung insbesondere aus medizinischer Sicht nicht angezeigt erscheint.

Bei einem weiteren bevorzugten Ausführungsbeispiel der erfindungsgemäßen Vorrichtung ist die Steuereinheit so ausgebildet, daß die Analyse des Strahlenganges im Auge und/oder die Analyse der Oberfläche des Auges quasi zeitgleich mit der Bearbeitung eines optischen Elements durch den Strahl der kohärenten Lichtquelle erfolgen kann. Durch diese Modifikation der Steuereinheit ist es möglich, während der Bearbeitung des optischen Elements, d. h. beispielsweise während der Bearbeitung der Cornea mit dem Chirurgielaserstrahl, eine "online"-Überprüfung des Strahlenganges im Auge, wie es derzeit durch die Operation modifiziert ist, und/oder der Oberfläche dieses Auges im aktuellen Zeitpunkt durchzuführen und in die weitere Operation bzw. Bearbeitung einfließen zu lassen. Damit ist es möglich, daß man während einer Laserbehandlung der Cornea zur Korrektur refraktiver Sehfehler des Auges ständig den aktuellen refraktiven Wert des gesamten Sehapparates des Auges mißt und bei gegebenen störenden Einflüssen auf die Behandlung online den Behandlungsablauf auf den Zielwert der Refraktion des Auges exakt einstellen kann. Durch diese Kombination der optischen Systeme eines Chirurgielasers zum Abtrag von Cornea-Gewebe bzw. Linsenmaterial und eines Aberrometers zur Wellenfrontanalyse bzw. der Oberfläche des Auges wird diese optimierte Behandlung möglich. Besonders vorteilhaft ist hierbei die vollständige Erfassung der refraktiven Werte (sphärozylindrische Aberrationen sowie Aberrationen höherer Ordnung) des gesamten optischen Apparates des Auges, welche in letzter Instanz repräsentativ sind für die Qualität des erzielten operativen Eingriffs. Durch die vollständige Erfassung online und ohne Unterbrechung des Behandlungsablaufes wird hierdurch eine hinsichtlich des zeitlichen Ablaufs und der erzielbaren Präzision optimierte Vorrichtung bereitgestellt.

Besonders bevorzugt ist es auch möglich, die Analyse des Strahlengangs bzw. der Oberfläche des Auges im Wechsel mit der Bearbeitung des optischen Elements durchzuführen und so abschnittsweise den Fortgang der Behandlung bzw. der Operation des optischen Systems, insbesondere der Cornea, zu erfassen. Es ist auch denkbar, die Abtastung und Analyse des Strahlengangs bzw. der Oberfläche des Auges zeitlich in die Bearbeitung durch den Chirurgielaserstrahl zu verzahnen. Dadurch ist eine kontinuierliche bzw. quasi-kontinuierliche Messung bei echter Geräteintegration (z. B. jede Sekunde) und fortlaufende Neuberechnung der Lasersteuerung möglich.

Die Aufgabe wird weiter gelöst durch ein erfindungsgemäßes Verfahren zur Korrektur von Sehfehlern eines Auges, wobei der Strahlengang des Auges mittels einer Wellenfrontanalyse ermittelt wird und ein ideales Linsensystem berechnet wird, das zu einer Korrektur der Sehfehler des Auges führen würde. Besonders bevorzugt wird dieses Verfahren unter Einsatz einer erfindungsgemäßen Vorrichtung angewendet. Bei diesem Verfahren steht für die Berechnung der Korrektur des optischen Systems zur Überführung in ein ideales optisches System die intraokulare Aberration des Strahlenganges zur Verfügung.

Besonders bevorzugt wird bei einem weiteren erfindungsgemäßen Verfahren zusätzlich die Topographie des Auges analysiert. Damit stehen in diesem Verfahren noch weitere Informationen über die Fehlsichtigkeit des Auges zur Verfügung, insbesondere über Aberrationen, unsymmetrische Zylinder- und Hornhaut-Irregularitäten.

Bei einem weiteren bevorzugten Verfahren wird das ideale optischen System auf der Basis der aus der Wellenfrontanalyse und/oder der aus der Topographieanalyse ermittelten Daten bereitgestellt. Besonders bevorzugt wird dafür nur ein Element aus diesem optischen System bereitgestellt. Auf diese Weise wird in einem weiteren Schritt das korrigierende Element oder die korrigierenden Elemente auf der Basis der kompletten Daten der Fehlsichtigkeit hergestellt. Dieses Vorgehen führt so zur vollständigen Korrektur der Fehlsichtigkeit.

Bei einem weiteren bevorzugten Verfahren werden Schußpositionen zur Herstellung des idealen optischen Systems mittels der aus der Wellenfrontanalyse und/oder aus der Topographieanalyse ermittelten Daten berechnet. Auf diese Weise kann vorteilhaft das Laser-Spot-Excimer-Verfahren zur Herstellung der einzelnen Elemente des optischen Systems genutzt werden. Die Schußpositionen werden je nach einzusetzenden Materialien und unter Berücksichtigung des Fertigungszeitaufwandes optimiert.

Bei einem weiteren Verfahren der vorliegenden Erfindung wird das alte optischen System des Auges zu dem berechneten idealen optischen System umgeformt. Hierzu werden entweder Elemente des alten optischen Systems direkt bearbeitet oder entsprechend korrigierte Elemente hergestellt und eingesetzt bzw. alte Elemente gegen neue Elemente ausgetauscht. Durch dieses Verfahren ist die Überführung des alten (fehlsichtigen) optischen Systems des Auges in ein (neues) ideales optischen System möglich. Besonders bevorzugt wird eine neue Linse bzw. eine ICL nach dem Spot-Scanning-Prinzip mit einem Excimerlaser hergestellt.

Besonders bevorzugt wird beim erfindungsgemäßen Verfahren eine Kontaktlinse bearbeitet, die bereits auf dem Auge des Patienten angeordnet ist. Der Patient trägt während der Messung bevorzugt bereits eine die Standard-Fehlsichtigkeiten korrigierende Kontaktlinse bzw. bei Normalsichtigkeit nur eine therapeutische Kontaktlinse, die besonders bevorzugt eine gute Haftung besitzt und konstante Abbildungseigenschaften ohne Dezentrierung aufweist. Nun wird die Ablation auf der standardisierten Kontaktlinse vorgenommen und damit nicht invasiv eine risikolose Korrektur der höheren Aberration des Auges erzielt. Dadurch wird für den Patienten risikolos mit hoher Behandlungssicherheit insbesondere höhere Aberrationen korrigierbar, um den optischen Apparat des Auges zu befähigen, das Auflösungsvermögen der Retina auszunutzen und ein Supervisus zur Verfügung zu haben, der dem des normalsichtigen Auges überlegen ist. Patienten können nun ohne Rücksicht auf die Eigenschaften ihrer eigenen Augen mit Hinblick auf Transmissionswerte bzw. Austrocknung des Tränenfilms einen bevorzugten Supervisus erwerben und Erfahrungen im alltäglichen Leben damit sammeln. Durch die Entfernung der Kontaktlinse ist die alte Seheigenschaft wieder hergestellt.

Bevorzugt wird hierfür auch eine therapeutische Kontaktlinse ohne refraktive Wirkung eingesetzt. Hierbei werden sämtliche Abbildungsfehler auf dieser Kontaktlinse mit dem Laser korrigiert.

Besonders bevorzugt werden Kontaktlinsen aus PMMA oder Kunststofflinsen genutzt, welche besonders bevorzugt einen geringeren Materialabtrag für den eingesetzten Laser, beispielsweise einen 193 nm ArF-Excimerlaser gegenüber der menschlichen Cornea aufweisen. Insbesondere bevorzugt sind auch alle weichen Kontaktlinsen, die aufgrund ihres hohen Wassergehalts nahezu die gleichen Ablationseigenschaften wie die Cornea zeigen. So können vorteilhaft vor der Behandlung für jegliches herstellungsseitig standardisiertes Kontaktlinsenmaterial die exakten Ablationsraten bestimmt werden und damit reproduzierbar eine gewünschte refraktive Korrektur auf dem Auge vorgenommen werden.

Die so simulierte Korrekturmöglichkeit kann für eine spätere echte Hornhautoperation vorbereitend dienen, oder es kann für eine vorbestimmte Zeit eine derartige Linse mit maßgeschneiderter Korrektur genutzt werden. Insbesondere bei harten Kontaktlinsen kann auch eine längerfristige Benutzung erfolgen. Hierbei wird vorteilhafterweise eine entsprechende Markierung für die Achslage aufgebracht, die beim Einsetzen berücksichtigt wird.

Bevorzugt umfaßt das optischen System als Elemente die Augenlinse und/oder eine Intraokularlinse und/oder die Cornea des Auges und/oder eine Kontaktlinse und/oder eine ICL und/oder mindestens ein Brillenglas. Mittels refraktiver Chirurgie kann beispielsweise die Cornea des Auges umgeformt werden, um die bestehende Fehlsichtigkeit zu korrigieren (z. Bsp. die Oberfläche der Cornea über die Photorefraktive Keratektomie, PRK, oder durch Ablation innerer Gewebeschichten der Cornea durch die Laser assisted in situ Keratomileusis, LASIK). Diese Elemente weisen nicht nur rotationsgeometrische Korrekturen auf, sondern individuelle Strukturen zur Korrektur der Fehlsichtigkeit der Patienten. So ist es möglich, Intraokularlinsen oder Kontaktlinsen, insbesondere ICL, herzustellen, die - einmal in das Linsensystem eingebracht - nicht nur wie bisher die Fehlsichtigkeit des Auges grob korrigieren, sondern darüber hinaus alle Irregularitäten, Unsymmetrien und Strahlverzerrungen mitkorrigieren. Damit kann ein visus erreicht werden, der über dem des normalen menschlichen Auge liegt. Außerdem ist es mit diesem Verfahren möglich, Brillengläser herzustellen, die ebenfalls alle Irregularitäten, Unsymmetrien und Strahlverzerrungen des fehlsichtigen Auges bzw. des alten optischen Systems mitkorrigieren.

Weiter wird die Aufgabe gelöst durch ein ideales optischen System, das nach einem erfindungsgemäßen Verfahren und/oder mittels einer erfindungsgemäßen Vorrichtungen hergestellt wurde, wobei das optischen System Elemente aus implantationsgerechten und/oder adhäsionsgerechten und/oder ablationsgeeigneten Werkstoffen, insbesondere Kunststoff oder Glas, umfaßt. Durch die Wahl dieser Werkstoffe des erfindungsgemäßen Linsensystems ist die Verträglichkeit beim Einsatz dieser Elemente gewährleistet. Solche Werkstoffe sind beispielsweise PMMA, Acryl, Silikon oder eine Kombination dieser Werkstoffe.

Bei einem weiteren Ausführungsbeispiel der vorliegenden Erfindung ist ein ideales optischen System vorgesehen, das Elemente umfaßt, die refraktive und/oder diffraktive Strukturen umfassen. Refraktive und/oder diffraktive Strukturen werden bisher nur in der Strahlformung verwendet. Ein Minilinsensystem lenkt und formt den eintretenden Strahl, um eine spezielle Strahlverteilung in der Zielebene zu erreichen. Der Einsatz derartiger refraktiver und/oder diffraktiver Strukturen auf einzelnen Elementen eines optischen Systems erlaubt die gezielte Korrektur von Sehschwächen in ungewöhnlich idealer Weise. So ist es durch den Einsatz dieser Strukturen möglich, einzelne nicht stetige Aberrationen zu korrigieren oder aber auch den optischen Systemen Eigenschaften zu verleihen, die ein normales menschliches Auge nicht aufweist.

Die Aufgabe der Erfindung wird weiter durch ein Element eines (idealen) Linsensystems gelöst, das refraktive und/oder diffraktive Strukturen aufweist. Solche Elemente können Intraokularlinsen, modifizierte Cornea, Kontaktlinsen, ICL oder Brillengläser sein.

Ausführungsbeispiele der Erfindung und vorteilhafte Ausgestaltungen sollen im Folgenden anhand von Zeichnungen näher erläutert werden. Dabei zeigen :
- Fig. 1: ein Blockschaltbild für ein Ausführungsbeispiel einer erfindungsgemäßen Vorrichtung zur Korrektur einer Aberration im Strahlengang eines Auges auf einer Linse 6 ;
- Fig. 2: ein Blockschaltbild für ein Ausführungsbeispiel einer erfindungsgemäßen Vorrichtung zur Korrektur einer Aberration im Strahlengang eines Auges, auf dem eine Linse 6 aufgebracht ist und
- Fig. 3: ein Blockschaltbild für ein Ausführungsbeispiel einer erfindungsgemäßen Vorrichtung zur Korrektur einer Aberration im Strahlengang eines Auges ohne Kontaktlinse.

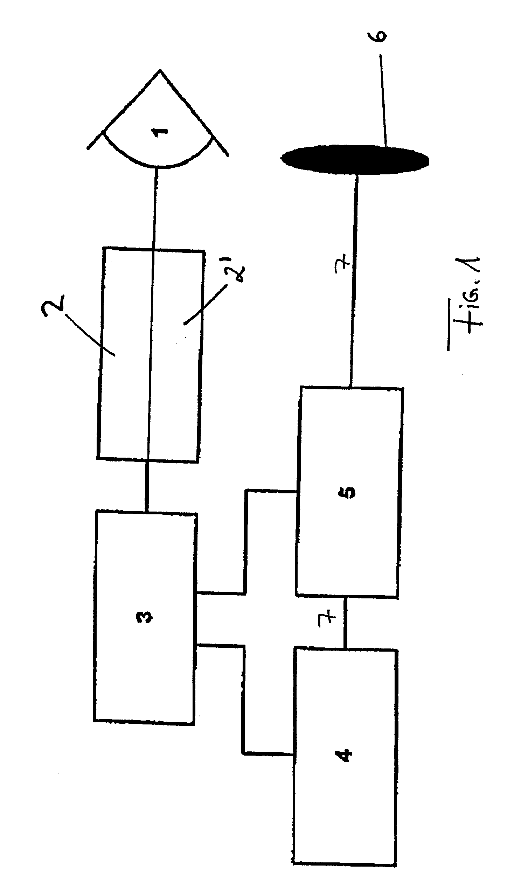

In Figur 1 ist ein Blockschaltbild für ein Ausführungsbei- spiel einer erfindungsgemässen Vorrichtung zur Korrektur von Sehfehlern eines Auges auf einer Linse dargestellt. Eine Wellenfrontanalyseeinheit 2 und eine Topographieanalyseeinheit 2' sind mit einer Steuereinheit 3 verbunden. Die Steuereinheit 3 ist über einen Bus mit einem Laser 4 und einer Strahlmodifikationseinrichtung 5 verbunden. Hinter der Strahlmodifikationseinrichtung 5 ist eine Linse 6 dargestellt. Vor der Wellenfrontanalyseeinheit 2 und der Topographieanalyseeinheit 2' ist ein Auge 1 dargestellt.

Im Betriebszustand tasten die Strahlen der Wellenfrontanalyseeinheit 2 und der Topographieanalyseeinheit 2' das Auge 1 ab und übermitteln die gewonnenen Signale an die Steuereinheit 3. Als Strahlen werden hier bevorzugt Strahlen einer kohärenten Lichtquelle eingesetzt, besonders bevorzugt Strahlen einer IR-Diode oder eines grünen Lasers. In der Steuereinheit 3 werden die Signale verarbeitet und das ideale optischen System für dieses Auge 1 berechnet. Im dargestellten Fall wird hier als Element des optischen Systems eine ideale Linse 6 berechnet. Insbesondere werden in der Steuereinheit 3 ausgehend von den aus den Signalen gewonnenen Daten unter Berücksichtigung der laserrelevanten Daten sämtliche Schußpositionen berechnet, die für den Laser 4 zur Herstellung der idealen Linse 6 benötigt werden. Die Steuereinheit 3 steuert daraufhin den Laser 4 an und bestimmt Energie und Pulsrate des chirurgischen Strahles 7. Der Strahl 7 wird durch die Strahlmodifikationseinrichtung 5 geleitet. In der Strahlmodifikationseinrichtung 5 wird der Strahl 7 gemäß den berechneten Schußpositionen durch die Vorgaben der Steuereinheit 3 über Scanner und Linsensysteme geformt und abgelenkt, so daß durch Ablation von Material auf der Rohlinse durch den gesteuerten chirurgischen Laserstrahl 7 die kundenspezifische Linse 6 hergestellt wird. Die Steuereinheit 3 kann auch bevorzugt in mehreren Teilsteuereinheiten ausgeführt sein, die mit einzelnen Bauteilen der Vorrichtung verbunden sein können.

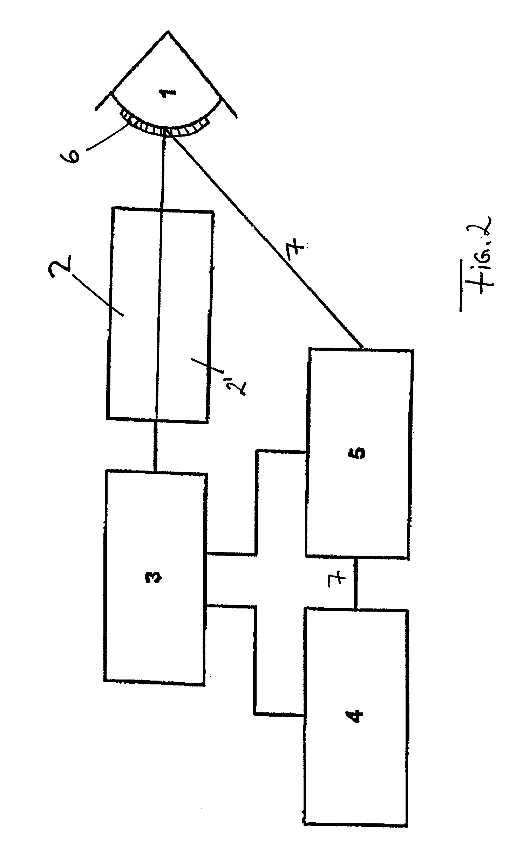

In Figur 2 ist ein Blockschaltbild für ein weiteres Ausführungsbeispiel einer erfindungsgemäßen Vorrichtung zur Korrektur einer Aberration im Strahlengang eines Auges, auf dem eine Linse aufgebracht ist, dargestellt. Im Prinzip entspricht der Aufbau dem der Figur 1 mit dem Unterschied, daß eine Kontaktlinse 6 direkt auf dem Auge 1 aufgebracht ist und dort in situ die Ablation vorgenommen wird. Hierzu wird der chirurgische Laserstrahl 7 von der Strahlenmodifikationseinrichtung 5, bevorzugt über weitere optische Element wie Spiegel (nicht dargestellt), auf die Linse 6 gerichtet, die direkt auf dem Auge 1 aufliegt. Die Ablation des Linsenmaterials findet nun in situ auf dem Auge statt, so daß nun durch die Analyseeinrichtung 2 bzw. 2' bevorzugt online parallel zur Ablation der Strahlverlauf im System des Auges 1 und der Linse 6 sowie die Oberfläche dieses Systems - hier also insbesondere der Linse 6 - analysiert werden kann und die Behandlung durch den Strahl 7 direkt überprüft und beurteilt werden kann. Die so hergestellte ideale Linse 6 vermittelt dem Interessenten nun einen Eindruck des kompletten optischen Systems sowie der Operationsbedingungen, ohne daß er sich einer irreversiblen Operation unterzogen hätte.

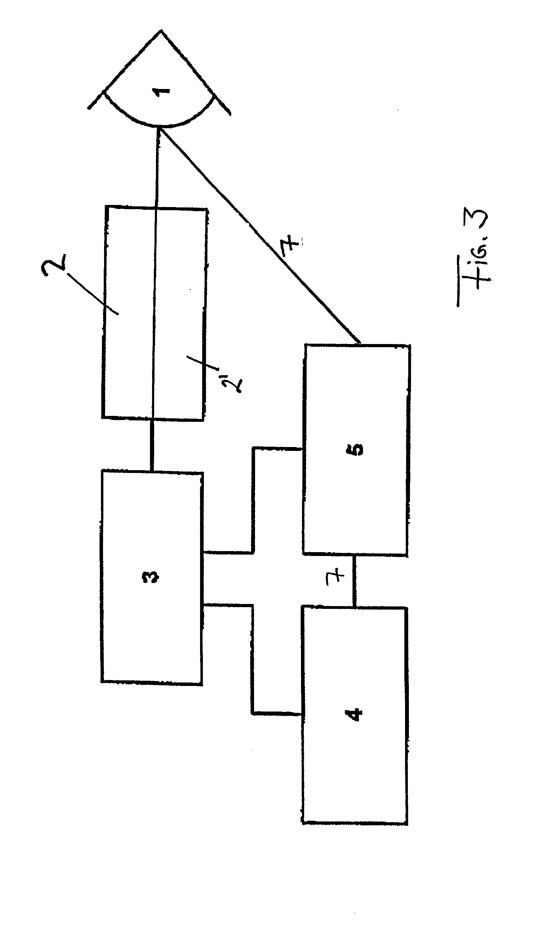

In Figur 3 ist ein Blockschaltbild für ein Ausführungsbei- spiel einer weiteren erfindungsgemäßen Vorrichtung zur Korrektur der Aberration im Strahlengang eines Auges ohne Kontaktlinse dargestellt. Hier kann nun durch den chirurgischen Strahl 7 bevorzugt zeitgleich mit der Analyse des Auges 1 durch die Analyseeinrichtung 2,2'der Fortgang der Operation online beobachtet werden und über die Steuereinrichtung 3 können während der Operation Nachberechnungen durchgeführt werden, so daß iterativ eine Behandlung des Auges 1 durch den jeweils auf die aktuellen Gegebenheiten reagierenden und eingesetzten Laserstrahl 7 erfolgen kann. Daneben kann über beispielsweise ein Mikroskop der Fortgang der Operation auch visuell beobachtet werden (nicht dargestellt).

Besonders bevorzugt wird auf der Retina des Auges 1 mit Hilfe einer Lichtquelle der Analyseeinrichtung 2,2' (Lichtquelle nicht gesondert dargestellt) ein Punkt projiziert. Dabei nutzt man einen nahezu parallelen externen Strahlengang und nutzt die fokussierende Wirkung des optischen Apparates des Auges, um einen möglichst kleinen Punkt erzeugen zu können. Die Intensitäten sind entsprechend der genutzten Wellenlänge und der Bestrahlungsdauer so gering, daß keinerlei Schaden auf der Retina entstehen kann, jedoch genügend reflektierte Intensität vorliegt.

Die reflektierte Welle faßt bei ihrem Durchgang durch das aberrationsbehaftete optische System des Auges alle Abbildungsfehler. Die entsprechende deformierte Wellenfront gelangt in die Wellenfront-Analyseeinrichtung 2 bzw. die Topographie-Analyseeinreinheit 2', von wo die Aberrationsdaten über eine entsprechende Elektronik bzw. Steuereinheit 3 bevorzugt einem Computer zugeführt werden. Mit Hilfe der Computersoftware wird aus den Aberrationsdaten jeweils ein aktuelles Ablationsprofil berechnet, welches den Excimerlaser 4 mit Spot-Scanning-System ansteuert und eine zielgesteuerte Behandlung über die Strahlenmodifikationseinrichtung 5 realisiert.

Eine solche Messung kann auch sporadisch durchgeführt werden, während beispielsweise 80 % der Behandlungszeit. Die Analyseeinrichtung 2 bzw. 2' können auf einem separaten Stand angeordnet werden, so daß Laser und Meßgerät abwechselnd einschwenken bzw. bevorzugt ist das Meßgerät im Laser integriert und mißt bei der Unterbrechung des Laserbeschusses.. Besonders bevorzugt wird eine Neuberechnung der Restbehandlung auf Anforderung des Bedieners durchgeführt. Diese Messung kann besonders bevorzugt auch kontinuierlich oder quasikontinuierlich bei Geräteintegration und fortlaufender Neuberechnung der Lasersteuerung durchgeführt werden.

Auf diese Weise ist ein neues und vorteilhaftes Verfahren und eine Vorrichtung zur vollständigen Korrektur von Sehfehlern des menschliches Auges angegeben worden. Es wurden Kombinationen von Meß- und Bearbeitungsverfahren angegeben, welche in ihrer erfindungsgemäßen Anwendung die vollständige Korrektur des menschlichen Auges ermöglichen. Dabei werden Meßverfahren eingesetzt, welche die Oberfläche der Cornea präzise erfassen können und auch die im weiteren Strahlengang bis zur Netzhaut entstehenden Abbildungsfehler registrieren. Die rechnergestützte Auswertung dieser Meßergebnisse ergibt in Verbindung mit der Berechnung ideal korrigierter Augenlinsen (beispielsweise nach Katarakt-Operationen) oder ideal korrigierender Corneaoberflächen die Möglichkeit, bevorzugt mit einem Spot-Scanning-Excimerlasersystem topographiegestützt eine patientenspezifische Linse herzustellen und/oder die Hornhaut ideal korrigierend zu formen.

Insbesondere kann die Korrektur über die Modifikation eines Elements des optischen Systems erfolgen. So reicht es zur Verbesserung des Sehvermögens eines Patienten mit grauem Star und einer Fehlsichtigkeit aus, die intraokulare Linse vollständig zu korrigieren. In einem solchen Fall ist es nicht mehr erforderlich, neben der Katarakt-Operation noch eine refraktive Operation durchzuführen.

Die Erfindung umfaßt insbesondere folgendes:
1. Vorrichtung zur Korrektur von insbesondere refraktiven Sehfehlern eines Auges (1), umfassend
   eine kohärente Lichtquelle (4),eine Strahlmodifikationseinrichtung (5) zur Formung und Ablenkung eines Strahles der kohärenten Lichtquelle (4)**dadurch gekennzeichnet, daß** eine Wellenfrontanalyseeinrichtung (2) zur Analyse einer Wellenfront des Strahlenganges im Auge (1) vorgesehen ist.
2. Vorrichtung nach Nr. 1, **dadurch gekennzeichnet, daß** zusätzlich eine Topographieanalyseeinheit (2') zur Analyse der Oberfläche des Auges (1) vorgesehen ist.
3. Vorrichtung nach Nr. 1 oder 2, **dadurch gekennzeichnet, daß** weiterhin eine Steuereinheit (3) zur Verarbeitung von Signalen der Wellenfrontanalyseneinheit (2) und/oder zur Verarbeitung von Signalen der Topographieanalyseeinheit (2'), und/oder zur Steuerung der kohärenten Lichtquelle (4) und/oder zur Steuerung der Strahlmodifikationseinrichtung (5) vorgesehen ist.
4. Vorrichtung nach einer der Nr. 1-3, **dadurch gekennzeichnet, daß** die Strahlmodifikationseinrichtung (5) so ausgebildet ist, daß mit dem Strahl eine Intraokularlinse und/oder eine Augenlinse und/oder die Cornea des Auges (1) und/oder eine Kontaktlinse und/oder eine Implantable Contact Lens und/oder ein Brillenglas bearbeitbar ist.
5. Vorrichtung nach einer der Nr. 1-4, **dadurch gekennzeichnet, daß** die kohärente Lichtquelle (4) ein Laser, insbesondere ein Spot-Scanning-Excimerlasersystem, ist.
6. Verfahren zur Korrektur insbesondere von refraktiven Sehfehlern eines Auges (1), insbesondere unter Einsatz einer Vorrichtung nach den vorhergehenden Ansprüchen, **dadurch gekennzeichnet, daß** der Strahlengang des Auges mittels Wellenfrontanalyse ermittelt wird; und daß ein ideales optisches System berechnet wird, das zu einer Korrektur der Sehfehler des Auges (1) führen würde.
7. Verfahren nach Nr. 6, **dadurch gekennzeichnet, daß** zusätzlich die Topographie des Auges (1) analysiert wird.
8. Verfahren nach einer der Nr. 6 oder 7, **dadurch gekennzeichnet, daß** das ideale optische System auf der Basis der aus der Wellenfrontanalyse und/oder der aus der Topographieanalyse ermittelten Daten bereitgestellt wird.
9. Verfahren nach einer der Nr. 6-8, **dadurch gekennzeichnet, daß** weiterhin Schußpositionen zur Herstellung des idealen optischen Systems mittels der aus der Wellenfrontanalyse und/oder aus der Topographieanalyse ermittelten Daten berechnet werden.
10. Verfahren nach einer der Nr. 6-9, **dadurch gekennzeichnet, daß** das alte optische System des Auges (1) zu dem berechneten idealen optischen System umgeformt wird.
11. Verfahren nach einer der Nr. 6-10, **dadurch gekennzeichnet, daß** das optische System die Augenlinse und/oder eine Intraokularlinse und/oder die Cornea des Auges und/oder eine Kontaktlinse und/oder eine ICL und/oder mindestens ein Brillenglas umfaßt.
12. Ideales optisches System, das nach einem Verfahren einer der Nr. 6-10 oder mit einer Vorrichtung nach einer der Nr. 1-5 hergestellt wurde, **dadurch gekennzeichnet, daß** das optische System Elemente aus implantationsgerechten und/oder adhäsionsgerechten und/oder ablationsgeeigneten Werkstoffen, insbesondere Kunststoff oder Glas, umfaßt.
13. Ideales optisches System nach Nr. 12, **dadurch gekennzeichnet, daß** das optische System Elemente mit refraktiven und/oder diffraktiven Strukturen umfaßt.
14. Element zur Verwendung in einem optischen System, **dadurch gekennzeichnet, daß** das Element refraktive und/oder diffraktive Strukturen aufweist.
15. Verwendung eines Verfahrens nach einer der Nr. 6-10 und/oder einer Vorrichtung nach einer der Nr. 1-5 zur vollständigen Korrektur eines Sehfehlers eines Auges.

## Patentansprüche

1. Vorrichtung zur Korrektur von insbesondere refraktiven Sehfehlern eines Auges (1), umfassend
- eine kohärente Lichtquelle (4),
- eine Strahlmodifikationseinrichtung (5) zur Formung und Ablenkung eines Strahles der kohärenten Lichtquelle (4), wobei die Strahlmodifikationseinrichtung (5) so ausgebildet ist, daß mit dem Strahl mindestens eines der Elemente aus folgender Gruppe bearbeitbar ist: eine Intraokularlinse, eine Augenlinse, die Cornea des Auges (1), eine Kontaktlinse, eine Implantable Contact Lens und ein Brillenglas,
- eine Wellenfrontanalyseeinrichtung (2) zur Analyse einer Wellenfront des Strahlenganges im Auge (1),
- eine Topographieanalyseeinheit (2') zur Analyse der Oberfläche des Auges (1), und
- eine Steuereinheit (3) zur Verarbeitung von Signalen der Wellenfrontanalyseeinrichtung (2) und von Signalen der Topographieanalyseeinheit (2') und zur Steuerung der kohärenten Lichtquelle (4) und/oder der Strahlmodifikationseinrichtung (5), um zu einer Korrektur von Sehfehlern des Auges (1) das Element der Gruppe zu bearbeiten.

2. Vorrichtung zur Korrektur von insbesondere refraktiven Sehfehlern eines Auges (1), umfassend
- eine kohärente Lichtquelle (4),
- eine Strahlmodifikationseinrichtung (5) zur Formung und Ablenkung eines Strahles der kohärenten Lichtquelle (4), wobei die Strahlmodifikationseinrichtung (5) so ausgebildet ist, daß mit dem Strahl mindestens eines der Elemente aus folgender Gruppe bearbeitbar ist: eine Intraokularlinse, eine Augenlinse, die Cornea des Auges (1), eine Kontaktlinse, eine Implantable Contact Lens und ein Brillenglas,
- eine Wellenfrontanalyseeinrichtung (2) zur Analyse einer Wellenfront des Strahlenganges im Auge (1) und
- eine Steuereinheit (3) zur Verarbeitung von Signalen der Wellenfrontanalyseeinrichtung (2) und von Signalen der Topographieanalyseeinheit (2') und zur Steuerung der kohärenten Lichtquelle (4) und der Strahlmodifikationseinrichtung (5), um zu einer Korrektur von Sehfehlern des Auges (1) das Element der Gruppe zu bearbeiten, wobei
- die Steuereinheit (3) aus den Signalen für eine Strahlmodifikation erforderliche Parameter bestimmt und diese zur Steuerung der kohärenten Lichtquelle benutzt und auch zur Steuerung der Strahlmodifikationseinrichtung (5) einsetzt, um eine Geometrie des Strahles im Element der Gruppe und über eine Ablenkung des Strahles einen Zielort im Element der Gruppe festzulegen.

3. Vorrichtung nach Anspruch 1 oder 2, wobei die kohärente Lichtquelle (4) ein Laser, insbesondere ein Spot-Scanning-Excimerlasersystem oder ein Femtosekundenlaser ist.

4. Vorrichtung nach Anspruch 1, wobei die Steuereinheit (3) die Signale der Wellenfrontanalyseeinrichtung (2) und die Signale der Topographieanalyseeinheit (2') getrennt verarbeitet und auswertet.

5. Vorrichtung nach Anspruch 2, wobei die Steuereinheit (3) Schußpositionen für die Abgabe gepulster Laserstrahlung berechnet.

6. Vorrichtung nach einem der obigen Ansprüche, wobei die Strahlmodifikationseinrichtung (5) ein den Strahl der kohärenten Lichtquelle (4) lenkendes und formendes Minilinsensystem aufweist, um eine spezielle Strahlverteilung in einer Zielebene zu erreichen.
